# EUROPEAN PATENT SPECIFICATION

(11) **EP 4 043 064 B1**
(45) Date of publication and mention of the grant of the patent: **09.09.2026**
(21) Application number: 22153481.1
(22) Date of filing: 26.01.2022
(51) Int. Cl.: A61N 1/02, A61N 1/36, A61N 1/378, A61F 11/00, H01F 27/00, H01F 38/00

(54) **MEDICAL DEVICE**
MEDIZINISCHE VORRICHTUNG
DISPOSITIF MÉDICAL

(30) Priority: 12.02.2021 EP 21156734
(43) Date of publication of application: 17.08.2022
(73) Proprietor: Oticon Medical A/S, 2765 Smørum (DK)
(72) Inventor: LORENZEN, Michael Rodas, DK-2765 Smørum (DK)
(74) Representative: Lind Edlund Kenamets Intellectual Property AB

(56) References cited:
- EP-A1- 3 441 111
- SE-A1- 1 951 275
- SE-A1- 1 951 276
- US-A1- 2010 106 225

## Description

### FIELD

The present disclosure relates to a medical device. More particularly, the disclosure relates to a medical device comprising: an implantable unit configured to be at least partly implanted in the body of a user; a transformer core configured to be arranged at least partly under the skin of the user; an internal cabling configured to connect the implantable unit to the transformer core, wherein in the internal cabling comprises a first winding around the transformer core; an external unit configured to be at least partly arranged outside the body of the user, the external unit comprising: a power supply circuity and a connector coupled to the power supply circuity configured to supply power from the power supply circuity to the implantable unit via the transformer core, wherein the connector comprises a first connector part and a second connector part.

### BACKGROUND

Any discussion of the prior art throughout the specification should in no way be considered as an admission that such prior art is widely known or forms parts of common general knowledge in the respective field.

Medical devices having one or more implantable units, generally referred to as implantable medical devices, have provided a wide range of benefits to users or patients over recent decades. In particular, devices such as implantable hearing aids, implantable pacemakers, defibrillators, eye implants, retina implants, heart pumps, drug delivery systems, gastric implants, nerve stimulators, brain stimulators, functional electrical stimulation devices, such as cochlear prostheses, organ assist or replacement devices, and other partially or completely-implanted medical devices, have been successful in performing life-saving and/or lifestyle enhancement functions for a number of years.

As such, the type of implantable devices and the range of functions performed thereby have increased over the years. For example, many such implantable medical devices often include one or more instruments, apparatus, sensors, processors, controllers or other functional mechanical, electrical or electronic components that are permanently or temporarily implanted in a patient to perform diagnosis, prevention, monitoring, treatment or management of a disease or injury or symptom thereof, or to investigate, replace or modify of the anatomy or of a physiological process. Many of these implantable components receive power and/or data over a wireless transcutaneous link from external units that are part of, or operate in conjunction with, the implantable unit.

Said wireless transcutaneous link is conventionally realized as an inductive link, with an external unit comprising a transmitter or first winding and an implantable unit comprising a receiver or second winding. Typically, the receiver winding is implanted below the skin and the transmitter winding is attached to the skin of the user such that the two windings are substantially in parallel planes on both sides (external and implantable positions) of the skin. These systems are typically referred as TET links (TET-Transcutaneous Energy Transfer). For such TET links it is rather difficult to fixate and position the transmitter winding to the skin of a patient. Gluing solutions and special vests to fixate/position the transmitter winding have been tried. Especially for life sustaining applications like heart pumps, ventricular assist devices or total artificial hearts this fixation/positioning is very critical. If the transmitter winding falls off or if it is in the wrong position the transcutaneous power transfer is affected which could in the worst case be life threatening for the patient or user.

Further, for a conventional winding arrangement like the TET links where the transmitter winding and the receiver winding are on opposite sides of the skin and in parallel planes, the coupling coefficient is low because most of the magnetic field lines that the transmitter winding generates are not picked up by the receiver winding, thus leading to poor energy transfer efficiency. In addition, as the two windings are located on opposite sides of the skin, any change in winding separation, for example by way of increase in skin thickness, may result in a rapid drop in the coupling coefficient between the two windings. In view of the efficiency problem, the external unit usually includes a relatively large battery compartment or multiple batteries so that the implantable medical device is useable for a usage period that doesn't cause annoyance for the user, for example requiring the user to frequently change or recharge the batteries. Besides making the external unit aesthetically less appealing, the additional weight of the battery compartment or multiple batteries also requires a stronger fixation/positioning that may possibly lead to discomfort and in extreme cases irritation or infection of the skin. Further, also when the power efficiency is low a conventional TET link results in major energy losses in the transmitter winding and/or receiver winding which may become too hot. For life sustaining applications like heart pumps and total artificial hearts where the TET link runs in a continuous mode, dissipated heat results in skin heating. The heating of the skin may cause skin necrosis i.e. skin cells may die. So-called skin tunnel transformers have been around to address these fixations, positioning and heating problems. Skin tunnel transformers are for example described in "IEEE Transactions on Bio-Medical Engineering, Vol. Bme-15, No. 4, October 1968". The skin tunnel transformer has the advantage of a very high energy power transfer efficiency and a good positioning/fixation mechanism for life sustaining medical implants. However, to create the skin tunnel transformer one needs to either cut a transformer core of the skin tunnel transformer in two parts to be able to mount it through the skin tunnel which will have a detrimental effect on the energy transfer efficiency or one needs to feed the transmitter winding wire several times through the skin tunnel to achieve the optimum energy power transfer efficiency which is extremely non-user friendly. For life sustaining medical devices, like heart pumps or similar, feeding the transmitter winding wire several times through the skin tunnel is unacceptable from a safety point of view. If the number of turns of the winding are made too few or too many or made in the wrong direction the user may die.

Therefore, there is a need to provide an implantable medical device that includes a wireless transcutaneous link where one or more of the above-mentioned shortcomings are addressed. Document SE 1 951 275 describes a medical system with a connector forming an external winding, and SE 1 951 276 describes a medical system, an external unit and a method of initiating operation of an external unit.

### SUMMARY OF THE INVENTION

The invention is defined in independent claim 1. Advantageous embodiments of the invention are defined in dependent claims 2-13.

### SUMMARY OF THE DISCLOSURE

According to an aspect, the medical device comprises an implantable unit configured to be at least partly implanted in the body of a user; a transformer core configured to be arranged at least partly under the skin of the user; an internal cabling configured to connect the implantable unit to the transformer core, wherein in the internal cabling comprises a first winding around the transformer core; and an external unit configured to be at least partly arranged outside the body of the user. The external unit may comprise a power supply circuity and a connector coupled to the power supply circuity configured to supply power from the power supply circuity to the implantable unit via the transformer core, wherein the connector comprises a first connector part and a second connector part. The medical device may further comprise a first conductor path conductively connecting the power supply circuity and the first connector part, a middle loop conductor path conductively connecting a first section of the second connector part and a second section of the second connector part, wherein the middle loop conductor path preferably at least partly extends in the first connector part; a final conductor path conductively connecting the first connector part and the power supply circuity; wherein the first connector part and the second connector part are joinable to conductively connect the first conductor path and the final conductor path via the middle loop conductor path in series and, wherein a second winding is formable around the transformer core by joining the first connector part and the second connector part.

By joining the first connector part and the second connector part a second winding is formable in a secure and reproductive manner preferably around at least a part of the implanted transformer core. Preferably, a hole is pierced in the skin of the user, wherein the first and/or the second connector part is movable through the hole pierced into the skin of the user. Via the first winding and the second winding a transcutaneous link may be formable.

Moving the first and/or the second connector part through a hole pierced into the skin of the user may lead to a second winding being formed around at least a part of the transformer core opposite to the first winding in a reliable manner. This may reduce the time needed during surgery to form a second winding around the transformer core and thus to connect the implantable unit to the external unit via the first and the second winding. Hereby, the risk of forming a faulty or unreliable connection between the first and the second winding may be reduced by removing potential sources of human error.

The first winding and the second winding may individually comprise a number of turns around the transformer core. The number of turns around the transformer core of the first winding may be the same as the number of turns around the transformer core of the second winding. Alternatively, the number of turns of the first and the second winding may differ to each other.

Preferably, the middle loop conductor path may at least partly extend in the first connector part. Hereby, when joining the first connector part with the second connector part after moving the first connector part or the second connector part through the through hole or the opening of the transformer core, a second winding may automatically be provided without the need of threading conductive paths manually around the transformer core during surgery. This may at least lead to one turn or at least one loop being formed by the second winding around the transformer core.

The medical device may comprise a second conductor path conductively connecting the first conductor path and the second connector part; a first loop conductor path conductively connecting a first section of the first connector part and a second section of the first connector part; a final loop conductor path conductively connecting a third section of the first connector part and a fourth section of the first connector part; and a third conductor path conductively connecting the second connector part to the final conductor path.

The medical device may comprise a first connector part and a second connector part which are joinable to conductively connect the second conductor path and the middle loop conductor path via the first loop conductor path in series and to conductively connect the middle loop conductor path and the third conductor path via the final loop conductor path in series. This allows for a further number of turns or loops being provided as the second winding around the transformer core when the first connector part is joined with the second transformer part. Thus, the efficiency of the energy transfer to the implantable unit may be further enhanced.

The first loop conductor path and the final loop conductor path may solely extend in the first connector part. The first conductor path and the final conductor path may solely extend in the first connector part. Aforementioned arrangement of the conductor paths may lead to a beneficial arrangement of conductor paths in the connector, in particular in the first and the second connector part.

The first connector part and/or the second connector part of the medical device may be joinable via a plug connection. A plug connection may enable the first connector part and the second connector part to be connected in a convenient manner during surgery while preventing the first connector part and the second connector part to be unintentionally released from each other which could result in severe medical consequences for the user.

The portions of the conductor paths extending in between the first connector part and the second connector part may be enclosed at least partially by a protective housing. The protective housing may be made of a silicone material or any kind of material which protects the portions of the conductor paths extending in between the first connector part and the second connector part with minimal reduction of the inductive coupling efficiency between the first and the second windings. Further, the first connector part and the second connector part may be enclosed at least partially by a protective housing.

The transformer core may be substantially circular and/or comprise a through hole or an opening. This enables the first connector part and/or the second connector part to be movable through the transformer core in a convenient way in order to form the second winding.

The transformer core may consist of a magnetic core of a ferromagnetic material, such as laminated iron, iron powder, or ferrite, around which wire may be wound around. The shape of the transformer core may be toroidal with a through-going hole in a center of the transformer core, wherein the magnetic core circumference the through hole.

The transformer core may be toroidal with a circular, elliptical, rectangular, square, polygonal shape, curved shape or any combination thereof. The transformer core may include a shape that is selected from a circular, elliptical, rectangular, square, polygonal shape, a curved shape or any combination thereof.

The medical device may be selected from a group consisting of one or more of a cochlear implant comprising an implantable electrode array configured to be positioned within a cochlea of the user, the electrode array being configured to deliver electrical charges in accordance with the output, an auditory transmodiolar implant comprising an implantable electrode array configured to be positioned within a modiolus of the user, the electrode array being configured to deliver electrical charges in accordance with the output, an auditory brainstem implant comprising an implantable electrode array configured to be implanted directly onto brainstem, the electrode array being configured to deliver the electrical charges in accordance with the output, a bone conduction hearing aid comprising an implantable vibrator configured to be attached to skull of the user, the vibrator being configured to generate vibrations in accordance with the output, a middle ear implant comprising a vibratory unit configured to attach to one of the bones of the middle ear and/or to one of the windows of the cochlea, the vibratory unit being configured to generate vibrations in accordance with the output, an artificial pacemaker comprising an electrode array configured to deliver electrical charges in accordance with the output; an implantable ventricular assist device (VAD) comprising a pump configured to be attached to a user's heart, the pump being configured to provide blood flow within user's body; an implantable drug delivery system comprising an implantable capsule comprising a drug and a pump that is configured to attach to the implantable capsule and release, through a pumping action, a predefined amount of drug from the capsule to the user's body; and an implantable brain computer interface system comprising an implantable sensor adapted to capture neural signals in response to brain activity.

One or more of the above paragraphs refers to use of a medical device or a combination thereof. For example, a cochlear implant may be used alone but may also be used in combination such as providing mechanical stimulation by way of bone conduction hearing aid at a first ear and an electrical stimulation by way of cochlear implant at a second ear of the user. In another example, the same user may be utilizing a cochlear implant as well as an implantable cardioverter defibrillator.

The medical device may be an implantable hearing aid system, wherein the external unit further comprises a housing and an earhook attached to the housing. The housing of the external unit may include at least a microphone, an electronic unit, a processing unit configured to process an audio signal generated based on an acoustic signal received by the at least microphone in order to improve or augment the hearing capability of the user of the implantable hearing aid system. The housing of the external unit may further comprise at least a part of the connector and the implantable unit may include other components of a hearing aid, such as the processing unit, a transducer, a rechargeable battery etc.

The housing of the external unit may have a top part which includes the interface to the earhook, and the housing may further comprise a bottom part which is opposite to the top part. The housing may have a first longitudinal axis along a longitudinal length from the top part to the bottom part, and the transformer core may have a second longitudinal axis along the length of the transformer core. The first longitudinal axis and the second longitudinal axis may not be parallel.

The housing may be a behind-the-ear hearing aid, and the earhook may be bend in a direction not parallel to the first longitudinal axis. When the external unit is arranged on the ear and the implantable unit below the skin of the ear, the earhook may be arranged through the through-going hole or opening of the transformer core. The earhook may provide an ideal placement of the external unit behind the ear while also providing a good position of the second winding relative to the first winding around the transformer core.

The housing may be arranged behind the ear or in the ear of the user while the earhook is fixed to the ear by a clamping force or via a piercing in the skin. Preferably, the piercing in the skin goes through the through hole of the transformer core.

The housing and the earhook may be connected via a flexible unit. The flexible unit is configured to apply the earhook into a first position and at least a second position when applying a force onto the earhook or the housing. In the first position, the external unit is not fastened onto the ear, and thereby, the user is able to remove the external unit from the ear. In the second position, the external unit is fastened to the ear by a clamping force between the housing and the earhook. Fastening the external unit to the ear does not mean that the external unit is permanently fastened to the ear. The user of the system may detach the external unit and the earhook from the ear. The detaching of the external unit may be done by changing the position of the earhook or by changing the position of the external unit.

The flexible unit may be provided by the earhook or part of the earhook being made of a resilient material, such as silicone, which causes the earhook to be moveable in a direction which makes it easier for the user to remove the earhook off the ear.

The flexible unit may include a rotating means which is configured to rotate the earhook or at least a part of the earhook between the first position and at least the second position.

The housing and the earhook may be merged, and the housing may be made of a first material and the earhook may be made of a second material, wherein the second material may be more flexible than the first material. The second material may allow the earhook to be moved into the first position or the second position while applying a force onto the earhook or the housing. In the second position the external unit is fastened to the ear by a clamping force between the housing and the earhook onto the ear. The clamping force may be provided by the flexible unit or the material which the earhook may be made of.

The power supply circuity may be arranged inside the housing. This allows for the power supply circuity to be protected from external influences which may cause damage to the power supply circuity of hinder the functionality of the power supply circuity.

The first connector part may at least partly be arranged inside the earhook, wherein the earhook may comprise a port, in particular a plug port, to connect the first connector part and the second connector part. Preferably, the second connector part may be plugged into the port of the earhook.

The second connector part may at least partly be arranged inside the earhook and wherein at least a part of the earhook may be configured to be inserted through the through hole of the transformer core. This allows for the first and the second connector part being arranged at least partly inside the earhook. Also the part of the earhook comprising the second connector part may be moved through the through hole of the transformer core and connected to the first connector part, preferably, by a plug connection in the earhook. Hereby, the second winding may be formed in a reliable and convenient manner.

The transformer core may be configured to be implanted fully or partially within a part on an ear of the user. This allows for the second winding to be formed via the first and the second connector parts in a reliable manner. Additionally, the transformer core may be arranged in the proximity of the hearing device or rather the housing of the hearing device.

The external unit may be a Behind-The-Ear hearing aid, an In-The-Ear hearing aid, or an earhook hearing aid.

The external unit may include a shielding unit configured to magnetically shield an electronic unit and other units of the external unit from unwanted external electromagnetic fields provided inter alia by the first winding, the second winding and/or the transformer core. The shielding unit may be a resonant element being provided within a near field of the transformer core, the first winding and/or the second winding. The resonant element may be connected to the ground potential through energy dissipating means to terminate and dissipate electromagnetic noise from at least a part of the transformer core, the first winding and/or the second winding. The resonant element may implement a notch filter filtering the electromagnetic radiation noise. The resonant element may be connected to the ground potential through a battery of the external unit.

The term "other units of the external unit" may include one or more of processing units, rechargeable battery, control units, communication units, RF antenna, other antenna types, speaker, microphone etc.

The implantable unit may be an implantable processor and/or an implantable stimulator that is configured to generate an output. The output may be configured to generate perceivable stimulation for the user. For example, such perceivable stimulation includes perception of sound in case of implantable hearing aids. For the implantable hearing aid system, the output may include a stimulation pulse (usually frequency specific) or signal for generating vibrational force (usually frequency specific). In an embodiment, the implantable unit comprises a power controller adapted to control utilization of power received at the first winding.

Data signals may be additionally transmitted via the transcutaneous link or rather via the first and the second winding which may include control signals, configuration signals, information signals and/or audio signals, and the implantable unit may comprise a transducer, such as a vibration-based transducer and/or an electrical stimulator, for generating, based on the audio signals, vibration and/or electrical stimulation, respectively. The vibration may be transmitted onto the skull of the user via a fixture or via a housing of the vibration. The electrical stimulation may be applied to the cochlea of the user via an electrode array which comprises a plurality of electrodes arranged within the cochlea of the user. The vibration-based transducer may be an electromagnetic based transducer, or a piezoelectric based transducer.

The internal cabling may comprise an implantable housing or an implantable cover which may be arranged below the skin of the ear of the user, in particular between the skin and the skull of the user.

When the transformer core is arranged below the skin of the user, in particular below the skin of the ear, the radius or diagonal of the through-going hole or opening of the transformer core may be partially or nearly parallel with the skin of the user, in particular with the skin of the ear, and the depth of the through-going hole or opening may be partially or nearly orthogonal to the skin of the user, in particular to the skin of the ear of the user.

The through-going hole or opening of the transformer core may have an inner side which is chamfered or partially chamfered. The purpose of the chamfered inner side is to guide the second winding of the connector of the external unit into an optimal position when joining the first connector part and the second connector part. When being in the optimal position, an optimal inductive connection may be provided between the first and the second winding. The chamfered inner side may be angled to an extent which directs the connector towards the center of the through-going hole of the transformer core.

The first winding and the second winding may form a transcutaneous link which may be an inductive link which may be bidirectional or unidirectional.

At least a part of the second winding and/or the transformer core may be arranged in at least one of the following parts of the ear; Helix, Antihelix, Scapha, Triangular fossa, Pinna, Auricular lobule (earlobe), and Concha. By arranging an implantable unit, or to be more specific, the second winding and the transformer ear, within the ear results in a significant reduction in distance between the first and second winding in comparison to arranging the implantable unit in the head, i.e. between the skull and skin, of a patient. The reduction in distance provides in a more efficient coupling between the first and the second winding.

The external unit may include a first RF antenna configured to communicate with a second RF antenna arranged within the implantable unit, and thereby data signals may be communicated via the communication between the first and the second RF antenna, and power signals may be transmitted by the first winding to the second winding. Hereby, the power signals and the data signals may be communicated by separate communication interfaces, such as the RF and inductive interface. The RF link between the first and the second RF antenna may be based on Bluetooth, Bluetooth Low Energy, or other kind of short-range communication protocols.

The external unit may be configured to communicate the power signals and the data signals via the windings in different communication modes, where in a first communication mode the data signal is transmitted to the implantable unit and in a second communication the power signal is transmitted to the implantable unit. In the respective communication modes a resonance frequency of the windings may be different and/or, the modulation of the power signals and the data signals may be different.

Throughout the specification, unless stated explicitly otherwise, different disclosed embodiments should be considered combinable.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 schematically illustrates a first embodiment of a medical device comprising a first and a second connector part;
FIG. 2 schematically illustrates a detail view of a first embodiment of a part of an external unit;
FIG. 3 schematically illustrates a detail view of a second embodiment of a part of an external unit;
FIG. 4 schematically illustrates an external unit of a second embodiment of a medical device being an implantable hearing aid system;
FIG. 5 schematically illustrates an external unit of a third embodiment of a medical device being an implantable hearing aid system;
FIG. 6 schematically shows the external unit depicted in FIG. 5 attached to the ear of a user;
FIG: 7 schematically shows the external unit; and
FIG: 8 schematically shows another example of the external unit.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include micro-electronic-mechanical systems (MEMS), integrated circuits (e.g. application specific), microprocessors, microcontrollers, digital signal processors (DSPs), field programmable gate arrays (FPGAs), programmable logic devices (PLDs), gated logic, discrete hardware circuits, printed circuit boards (PCB) (e.g. flexible PCBs), and other suitable hardware configured to perform the various functionality described throughout this disclosure, e.g. sensors, e.g. for sensing and/or registering physical properties of the environment, the device, the user, etc. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

The medical device may be a hearing device (or hearing instrument, hearing assistance device) which may be or include a hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. 'Improving or augmenting the hearing capability of a user' may include compensating for an individual user's specific hearing loss. The "hearing device" may further refer to a device such as a hearable, an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of the middle ear of the user or electric signals transferred directly or indirectly to the cochlear nerve and/or to the auditory cortex of the user.

A "hearing system" refers to a system comprising one or two hearing devices, and a "binaural hearing system" or a "bimodal hearing system" refers to a system comprising two hearing devices where the devices are adapted to cooperatively provide audible signals to both of the user's ears either by acoustic stimulation only, acoustic and mechanical stimulation, mechanical stimulation only, acoustic and electrical stimulation, mechanical and electrical stimulation or only electrical stimulation. The hearing system, the binaural hearing system or the bimodal hearing system may further include one or more auxiliary device(s) that communicates with at least one hearing device, the auxiliary device affecting the operation of the hearing devices and/or benefitting from the functioning of the hearing devices. A wired or wireless communication link between the at least one hearing device and the auxiliary device is established that allows for exchanging information (e.g. control and status signals, possibly audio signals) between the at least one hearing device and the auxiliary device. Such auxiliary devices may include at least one of a remote control, a remote microphone, an audio gateway device, a wireless communication device, e.g. a mobile phone (such as a smartphone) or a tablet or another device, e.g. comprising a graphical interface, a public-address system, a car audio system or a music player, or a combination thereof. The audio gateway may be adapted to receive a multitude of audio signals such as from an entertainment device like a TV or a music player, a telephone apparatus like a mobile telephone or a computer, e.g. a PC. The auxiliary device may further be adapted to (e.g. allow a user to) select and/or combine an appropriate one of the received audio signals (or combination of signals) for transmission to the at least one hearing device. The remote control is adapted to control functionality and/or operation of the at least one hearing device. The function of the remote control may be implemented in a smartphone or other (e.g. portable) electronic device, the smartphone / electronic device possibly running an application (APP) that controls functionality of the at least one hearing device.

In general, a hearing device includes i) an input unit such as a microphone for receiving an acoustic signal from a user's surroundings and providing a corresponding input audio signal, and/or ii) a receiving unit for electronically receiving an input audio signal. The hearing device further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to (relatively) enhance a target acoustic source among a multitude of acoustic sources in the user's environment and/or to attenuate other sources (e.g. noise). In one aspect, the directional system is adapted to detect (such as adaptively detect) from which direction a particular part of the microphone signal originates. This may be achieved by using conventionally known methods. The signal processing unit may include an amplifier that is adapted to apply a frequency dependent gain to the input audio signal. The signal processing unit may further be adapted to provide other relevant functionality such as compression, noise reduction, etc. The output unit may include an output transducer such as a loudspeaker/receiver for providing an air-borne acoustic signal to the ear of the user, a mechanical stimulation applied transcutaneously or percutaneously to the skull bone, an electrical stimulation applied to auditory nerve fibers of a cochlea of the user. In some hearing devices, the output unit may include one or more output electrodes for providing the electrical stimulations such as in a Cochlear Implant, or the output unit may include one or more vibrators for providing the mechanical stimulation to the skull bone.

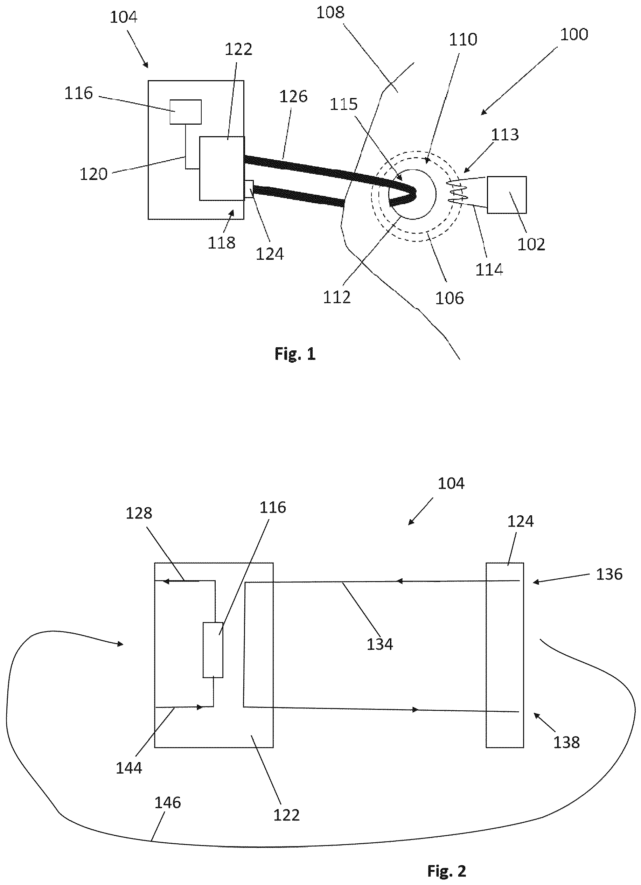

Now referring to FIG. 1, which schematically illustrates a first embodiment of a medical device 100 comprising a wireless transcutaneous link. The medical device 100 comprises an implantable unit 102 configured to be at least partly implanted in the body of a user. Furthermore, the medical device comprises an external unit 104 configured to be at least partly arranged outside the body of a user. Additionally, the medical device 100 comprises a transformer core 106 which is arranged at least partly under the skin 108 of the user.

The transformer core 106 is substantially circular and comprises a substantially circular through hole 110. Additionally, a hole 112 is pierced into the skin 108, wherein the pierced hole 112 in the skin 108 goes through the through hole 110 of the transformer core 106.

The implantable or internal unit 102 is connected via an internal cabling 114 to the transformer core 106. The internal cabling 114 comprises a first winding 113 around the transformer core 106.

The external unit 104 of the medical device 100 comprises a power supply circuity 116 and a connector 118 coupled to the power supply circuity 116 via a conductor path 120. The connector 118 comprises a first connector part 122 and a second connector part 124. The first connector part 122 and the second connector part 124 are conductively connected to each other via at least one conductor path. The at least one conductor path preferably is enclosed by a protective cover or a protective housing 126. The second connector part 124 is movable through the pierced hole 112 in the skin 108 and thus also through the through hole 110 of the transformer core 106, whereby a second winding 115 is formable around the transformer core 106 in a reliable and convenient manner.

FIG. 2 schematically illustrates a detail view of a first embodiment of a part of an external unit 104. As described in FIG. 1 the external unit 104 comprises a power supply circuity 116, a first connector part 122 and a second connector part 124. The external unit 104 comprises a first conductor path 128 for conductively connecting the power supply circuity 116 to the first connector part 122. Additionally a middle loop conductor path 134 conductively connects a first section 136 and a second section 138 of the second connector part 124, wherein the middle loop conductor path 134 partly extends in the first connector part 122. Additionally, a final conductor path 144 conductively connects the first connector part 122 and the power supply circuity 116. When joining the first connector part 122 and the second connector part 124 along arrow 146 through the through hole 110 of the transformer core 106, a second winding 115 is formable around the transformer core 106. This embodiment allows for at least one loop being formed around the transformer core 106 via the second winding 115. In particular, the first conductor path 128 is connected via the first portion 136 to middle loop conductor path 134 and the middle loop conductor path 134 is connected via the second portion 138 to the final conductor path 144.

Connecting the first connector part 122 and the second connector part 124 to each other and activating a voltage of the power supply circuity 116 leads to a time varying magnetic flux being formed in the transformer core 106 via the second winding 115 transferring energy to the internal unit 102 via the internal winding 114.

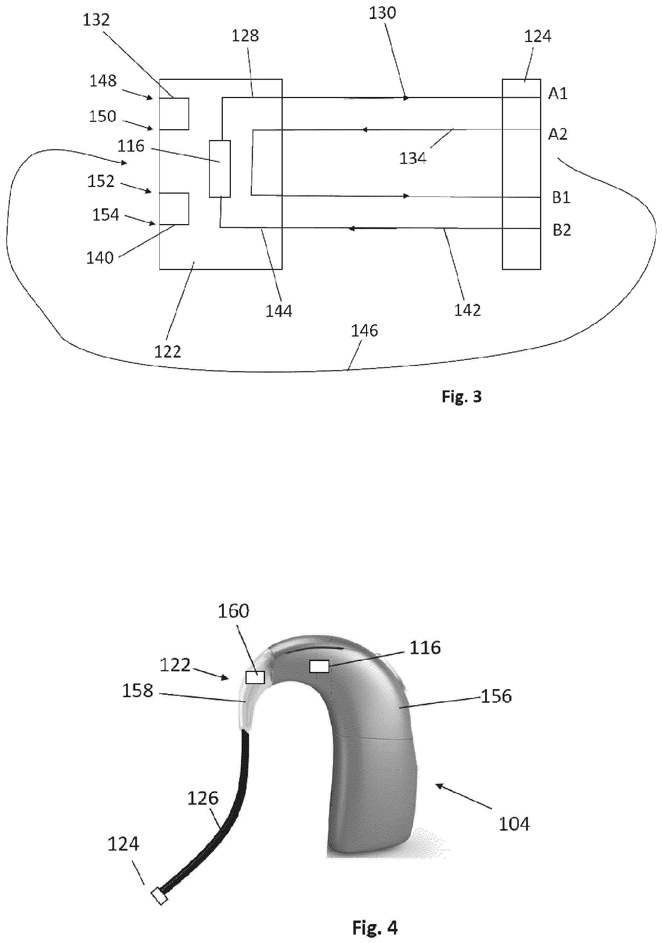

FIG. 3 schematically illustrates a detail view of a second embodiment of a part of an external unit 104. Differing from the first embodiment of a part of the external unit 104 depicted in FIG. 2, the medical device 100 further comprises a second conductor path 130 conductively connecting the first conductor path 128 and the second connector part 124; a first loop conductor path 132 conductively connecting a first section 148 of the first connector part 122 and a second section 150 of the first connector part 122; a final loop conductor path 140 conductively connecting a third section 152 of the first connector part 122 and a fourth section 154 of the first connector part 122; and a third conductor path 142 conductively connecting the second connector part 124 to the final conductor path 144.

When joining the first connector part 122 and the second connector part 124 along arrow 146 through the through hole 110 of the transformer core 106, a second winding 115 is formable around the transformer core 106. This embodiment allows at least one more loop being formed around the transformer core 106 via the second winding 115 in comparison to the first embodiment depicted in FIG. 2.

Connecting the first connector part 122 to the second connector part 124 leads to a conductive connection between the second conductor path 130 and the middle loop conductor path 134 via the first loop conductor path 132 and to a conductive connection between the middle loop conductor path 134 and the third conductor path 142 via the final loop conductor path 140.

FIG. 4 schematically illustrates a second embodiment of a medical device 100 being an implantable hearing aid system, wherein the external unit 104 comprises a housing 156 and an earhook 158 attached to the housing 156. The power supply circuity 116 is arranged inside the housing 156 of the implantable hearing aid system. The first connector part 122 is arranged inside the earhook 158 and conductively connected to the second connector part 124 as displayed in FIG. 2 or 3. The earhook 158 comprises a plug port 160 in which the second connector part 124 may be plugged in order to connect the first connector part 122 to the second connector part 124. The portions in between the first connector part 122 and the second connector part 124 are enclosed by a protective cover or housing 126.

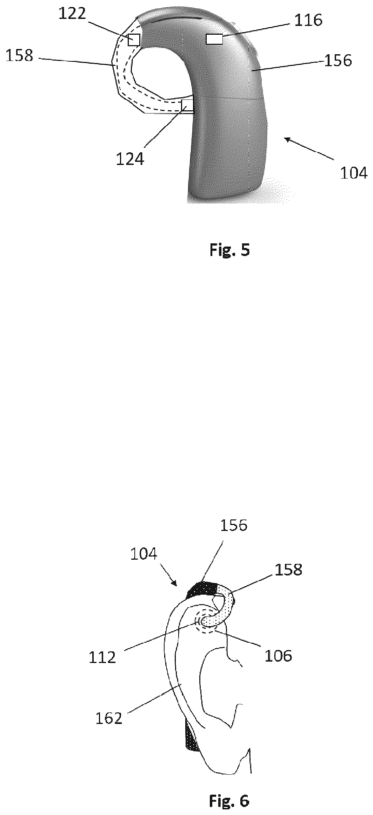

FIG. 5 schematically illustrates a third embodiment of a medical device 100 being an implantable hearing aid system. Differing from the second embodiment of a medical device 100 being illustrated in FIG. 4, the second connector part 124 is also arranged inside the earhook 158. As shown in FIG. 6, the earhook 158 may be inserted through the pierced hole 112 in the skin 108 of an ear 162 of the user and thus also through the through hole 110 of the transformer core 106, whereby a second winding 115 is formable around the transformer core 106 in a reliable and convenient manner. In order to connect the second connector part 124 with the first connector part 122, the tip of the earhook 158 may be plugged into a plug port 160.

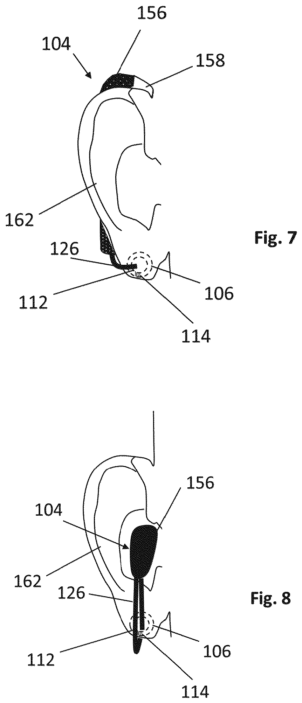

FIG. 7 shows the external unit 104 comprising a housing 156 and an earhook 158 attached to the housing 156. The power supply circuity 116 is arranged inside the housing 156 of the implantable hearing aid system. The first connector part 122 is arranged inside the housing 156 and conductively connected to the second connector part 124 as displayed in FIG. 2 or 3. The housing 156 comprises a plug port 160 in which the second connector part 124 may be plugged in order to connect the first connector part 122 to the second connector part 124. The portions in between the first connector part 122 and the second connector part 124 are enclosed by a protective cover or housing 126. In this present example, the plug port 160 and the first connector part are arranged in vicinity to a battery of the external 104 or to a lower part of the external unit 104. The pierced hole 112 is in the skin of an ear flip of the ear (i.e. lobule). FIG. 8 illustrates a similar example where the external unit 104 is an in-the-ear hearing aid.

### A computer program:

A computer program (product) comprising instructions which, when the program is executed by a computer, cause the computer to carry out (steps of) the method described above, in the 'detailed description of embodiments' and in the claims is furthermore provided by the present application.

### A computer readable medium

In an aspect, the functions may be stored on or encoded as one or more instructions or code on a tangible computer-readable medium. The computer readable medium includes computer storage media adapted to store a computer program comprising program codes, which when run on a processing system causes the data processing system to perform at least some (such as a majority or all) of the steps of the method described above.

By way of example, and not limitation, such computer-readable media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Combinations of the above should also be included within the scope of computer-readable media. In addition to being stored on a tangible medium, the computer program can also be transmitted via a transmission medium such as a wired or wireless link or a network, e.g. the Internet, and loaded into a data processing system for being executed at a location different from that of the tangible medium.

### A data processing system

In an aspect, a data processing system comprising a processor adapted to execute the computer program for causing the processor to perform at least some (such as a majority or all) of the steps of the method described above.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects. Reference to an element in the singular is not intended to mean "one and only one" unless specifically so stated, but rather "one or more". Unless specifically stated otherwise, the term "some" refers to one or more.

Accordingly, the scope of the invention is defined by the claims that follow.

## Claims

1. Implantable hearing aid system comprising:
• an implantable unit (102) configured to be at least partly implanted in the body of a user;
• a transformer core (106) configured to be arranged at least partly under the skin of the user, wherein the transformer core (106) is configured to be implanted fully or partially within a part of an ear (162) of the user;
• an internal cabling (114) configured to connect the implantable unit (102) to the transformer core (106), wherein the internal cabling (114) comprises a first winding (113) around the transformer core (106); and
• an external unit (104) configured to be at least partly arranged outside the body of the user, wherein the external unit (104) is a Behind-The-Ear hearing aid, an In-The-Ear hearing aid, or an earhook hearing aid, the external unit (104) comprising:
▪ a housing (156) and an earhook (158) attached to the housing (156);
▪ a power supply circuity (116) and
▪ a connector (118) coupled to the power supply circuity (116) configured to supply power from the power supply circuity (116) to the implantable unit (102) via the transformer core (106), wherein the connector (118) comprises a first connector part (122) and a second connector part (124);
wherein the system further comprises
• a first conductor path (128) conductively connecting the power supply circuity (116) and the first connector part (122);
• a second winding (115) is formable around the transformer core (106) by joining the first connector part (122) and the second connector part (124);
**characterized in that** the system further comprises
• a middle loop conductor path (134) conductively connecting a first section (136) of the second connector part (124) and a second section (138) of the second connector part (124), wherein the middle loop conductor path (134) preferably at least partly extends in the first connector part (122);
• a final conductor path (144) conductively connecting the first connector part (122) and the power supply circuity (116); and
• the first connector part (122) and the second connector part (124) are joinable to conductively connect the first conductor path (128) and the final conductor path (144) via the middle loop conductor path (134) in series.

2. Implantable hearing aid system according to claim 1, further comprising:
• a second conductor path (130) conductively connecting the first conductor path (128) and the second connector part (124);
• a first loop conductor path (132) conductively connecting a first section (148) of the first connector part (122) and a second section (150) of the first connector part (122);
• a final loop conductor path (140) conductively connecting a third section (152) of the first connector part (122) and a fourth section (154) of the first connector part (122); and
• a third conductor path (142) conductively connecting the second connector part (124) to the final conductor path (144).

3. Implantable hearing aid system according to claim 2, wherein the first connector part (122) and the second connector part (124) are joinable to conductively connect the second conductor path (130) and the middle loop conductor path (134) via the first loop conductor path (132) in series and to conductively connect the middle loop conductor path (134) and the third conductor path (142) via the final loop conductor path (140) in series.

4. Implantable hearing aid system according to claim 2 or 3, wherein the first loop conductor path (132) and the final loop conductor path (140) solely extend in the first connector part (122).

5. Implantable hearing aid system according to any of the claims 1 to 4, wherein the first conductor path (128) and the final conductor path (144) solely extend in the first connector part (122).

6. Implantable hearing aid system according to any of the claims 1 to 5, wherein the first connector part (122) and/or the second connector part (124) are joinable via a plug connection.

7. Implantable hearing aid system according to any of the claims 1 to 6, wherein the portions of the conductor paths (130, 134, 142) extending in between the first connector part (122) and the second connector part (124) are enclosed at least partially by a protective housing (126).

8. Implantable hearing aid system according to any of the claims 1 to 7, wherein the transformer core (106) is substantially circular and/or comprises a through hole (110).

9. Implantable hearing aid system according to any of the claims 1 to 8, wherein the implantable unit (102) is configured to generate an output and is selected from a group consisting of one or more of
• a cochlear implant comprising an implantable electrode array configured to be positioned within a cochlea of the user, the electrode array being configured to deliver electrical charges in accordance with the output,
• an auditory transmodiolar implant comprising an implantable electrode array configured to be positioned within a modiolus of the user, the electrode array being configured to deliver electrical charges in accordance with the output,
• an auditory brainstem implant comprising an implantable electrode array configured to be implanted directly onto brainstem, the electrode array being configured to deliver the electrical charges in accordance with the output,
• a bone conduction hearing aid comprising an implantable vibrator configured to be attached to skull of the user, the vibrator being configured to generate vibrations in accordance with the output, and
• a middle ear implant comprising a vibratory unit configured to attach to one of the bones of the middle ear and/or to one of the windows of the cochlea, the vibratory unit being configured to generate vibrations in accordance with the output.

10. Implantable hearing aid system according to any of the claims 1 to 9, wherein the power supply circuity (116) is arranged inside the housing (126).

11. Implantable hearing aid system according to claim 9 or 10, wherein the first connector part (122) is at least partly arranged inside the earhook (158) and wherein the earhook (158) comprises a port (160), in particular a plug port, to connect the first connector part (122) and the second connector part (124).

12. Implantable hearing aid system according to claim 8 and 11, wherein the second connector part (124) is at least partly arranged inside the earhook (158) and wherein at least a part of the earhook (158) is configured to be inserted through the through hole (110) of the transformer core (106).

## Patentansprüche

1. Implantierbares Hörgerätesystem, welches Folgendes umfasst:
- eine implantierbare Einheit (102), die dazu konfiguriert ist, zumindest teilweise in den Körper eines Benutzers implantiert zu werden;
- einen Transformatorkern (106), der dazu konfiguriert ist, zumindest teilweise unter der Haut des Benutzers angeordnet zu werden, wobei der Transformatorkern (106) dazu konfiguriert ist, vollständig oder teilweise innerhalb eines Teils eines Ohrs (162) des Benutzers implantiert zu werden;
- eine interne Verkabelung (114), die zum Verbinden der implantierbaren Einheit (102) mit dem Transformatorkern (106) konfiguriert ist, wobei die interne Verkabelung (114) eine erste Wicklung (113) um den Transformatorkern (106) herum umfasst; und
- eine externe Einheit (104), die dazu konfiguriert ist, zumindest teilweise außerhalb des Körpers des Benutzers angeordnet zu werden, wobei es sich bei der externen Einheit (104) um ein Hinter-dem-Ohr-Hörgerät, ein Im-Ohr-Hörgerät oder ein Ohrbügel-Hörgerät handelt, wobei die externe Einheit (104) Folgendes umfasst:
- ein Gehäuse (156) und einen Ohrbügel (158), der am Gehäuse (156) befestigt ist;
- einen Stromversorgungsschaltkreis (116); und
- ein Verbindungselement (118), das an den Stromversorgungsschaltkreis (116) gekoppelt ist, welches zum Bereitstellen von Strom vom Stromversorgungsschaltkreis (116) an die implantierbare Einheit (102) über den Transformatorkern (106) konfiguriert ist, wobei das Verbindungselement (118) ein erstes Verbindungsteil (122) und ein zweites Verbindungsteil (124) umfasst;
wobei das System ferner Folgendes umfasst:
- eine erste Leiterbahn (128), welche den Stromversorgungsschaltkreis (116) und das erste Verbindungsteil (122) leitend verbindet;
- eine zweite Wicklung (115), welche um den Transformatorkern (106) herum ausgebildet werden kann, indem das erste Verbindungsteil (122) und das zweite Verbindungsteil (124) miteinander verbunden werden;
**dadurch gekennzeichnet, dass** das System ferner Folgendes umfasst:
- eine mittlere Schleifenleiterbahn (134), welche einen ersten Abschnitt (136) des zweiten Verbindungsteils (124) und einen zweiten Abschnitt (138) des zweiten Verbindungsteils (124) leitend verbindet, wobei sich die mittlere Schleifenleiterbahn (134) vorzugsweise zumindest teilweise in das erste Verbindungsteil (122) erstreckt; und
- eine letzte Leiterbahn (144), welche das erste Verbindungsteil (122) und den Stromversorgungsschaltkreis (116) leitend verbindet; wobei
- das erste Verbindungsteil (122) und das zweite Verbindungsteil (124) miteinander verbunden werden können, um die erste Leiterbahn (128) und die letzte Leiterbahn (144) über die mittlere Schleifenleiterbahn (134) leitend in Reihe zu schalten.

2. Implantierbares Hörgerätesystem nach Anspruch 1, welches ferner Folgendes umfasst:
- eine zweite Leiterbahn (130), welche die erste Leiterbahn (128) und das zweite Verbindungsteil (124) leitend verbindet;
- eine erste Schleifenleiterbahn (132), welche einen ersten Abschnitt (148) des ersten Verbindungsteils (122) und einen zweiten Abschnitt (150) des ersten Verbindungsteils (122) leitend verbindet;
- eine letzte Schleifenleiterbahn (140), welche einen dritten Abschnitt (152) des ersten Verbindungsteils (122) und einen vierten Abschnitt (154) des ersten Verbindungsteils (122) leitend verbindet; und
- eine dritte Leiterbahn (142), welche das zweite Verbindungsteil (124) leitend mit der letzten Leiterbahn (144) verbindet.

3. Implantierbares Hörgerätesystem nach Anspruch 2, wobei das erste Verbindungsteil (122) und das zweite Verbindungsteil (124) miteinander verbunden werden können, um die zweite Leiterbahn (130) und die mittlere Schleifenleiterbahn (134) über die erste Schleifenleiterbahn (132) leitend in Reihe zu schalten und die mittlere Schleifenleiterbahn (134) und die dritte Leiterbahn (142) über die letzte Schleifenleiterbahn (140) leitend in Reihe zu schalten.

4. Implantierbares Hörgerätesystem nach Anspruch 2 oder 3, wobei sich die erste Schleifenleiterbahn (132) und die letzte Schleifenleiterbahn (140) lediglich in das erste Verbindungsteil (122) erstrecken.

5. Implantierbares Hörgerätesystem nach einem der Ansprüche 1 bis 4, wobei sich die erste Leiterbahn (128) und die letzte Leiterbahn (144) lediglich in das erste Verbindungsteil (122) erstrecken.

6. Implantierbares Hörgerätesystem nach einem der Ansprüche 1 bis 5, wobei das erste Verbindungsteil (122) und/oder das zweite Verbindungsteil (124) über eine Steckverbindung miteinander verbunden werden können.

7. Implantierbares Hörgerätesystem nach einem der Ansprüche 1 bis 6, wobei die Abschnitte der Leiterbahnen (130, 134, 142), die sich zwischen dem ersten Verbindungsteil (122) und dem zweiten Verbindungsteil (124) erstrecken, zumindest teilweise durch ein Schutzgehäuse (126) umschlossen sind.

8. Implantierbares Hörgerätesystem nach einem der Ansprüche 1 bis 7, wobei der Transformatorkern (106) im Wesentlichen kreisförmig ist und/oder ein Durchgangsloch (110) umfasst.

9. Implantierbares Hörgerätesystem nach einem der Ansprüche 1 bis 8, wobei die implantierbare Einheit (102) zum Erzeugen einer Ausgabe konfiguriert ist und aus einer Gruppe bestehend aus einem oder mehreren der Folgenden ausgewählt ist:
- ein Cochlea-Implantat, das eine implantierbare Elektrodenanordnung umfasst, die dazu konfiguriert ist, innerhalb einer Cochlea des Benutzers positioniert zu werden, wobei die Elektrodenanordnung zum Abgeben elektrischer Ladungen in Übereinstimmung mit der Ausgabe konfiguriert ist,
- ein transmodioläres Hörimplantat, das eine implantierbare Elektrodenanordnung umfasst, die dazu konfiguriert ist, innerhalb eines Modiolus des Benutzers positioniert zu werden, wobei die Elektrodenanordnung zum Abgeben elektrischer Ladungen in Übereinstimmung mit der Ausgabe konfiguriert ist,
- ein Hirnstamm-Hörimplantat, das eine implantierbare Elektrodenanordnung umfasst, die dazu konfiguriert ist, direkt an den Hirnstamm implantiert zu werden, wobei die Elektrodenanordnung zum Abgeben elektrischer Ladungen in Übereinstimmung mit der Ausgabe konfiguriert ist,
- ein Knochenleitungshörgerät, das einen implantierbaren Vibrator umfasst, der dazu konfiguriert ist, am Schädel des Benutzers angebracht zu werden, wobei der Vibrator zum Erzeugen von Schwingungen in Übereinstimmung mit der Ausgabe konfiguriert ist, und
- ein Mittelohrimplantat, das eine Schwingungseinheit umfasst, die dazu konfiguriert ist, an einem der Mittelohrknochen und/oder an einem der Fenster der Cochlea angebracht zu werden, wobei die Schwingungseinheit zum Erzeugen von Schwingungen in Übereinstimmung mit der Ausgabe konfiguriert ist.

10. Implantierbares Hörgerätesystem nach einem der Ansprüche 1 bis 9, wobei der Stromversorgungsschaltkreis (116) im Inneren des Gehäuses (126) angeordnet ist.

11. Implantierbares Hörgerätesystem nach Anspruch 9 oder 10, wobei das erste Verbindungsteil (122) zumindest teilweise im Inneren des Ohrbügels (158) angeordnet ist und wobei der Ohrbügel (158) einen Anschluss (160), insbesondere einen Steckanschluss, zum Verbinden des ersten Verbindungsteils (122) und des zweiten Verbindungsteils (124) umfasst.

12. Implantierbares Hörgerätesystem nach Anspruch 8 und 11, wobei das zweite Verbindungsteil (124) zumindest teilweise im Inneren des Ohrbügels (158) angeordnet ist und wobei zumindest ein Teil des Ohrbügels (158) dazu konfiguriert ist, durch das Durchgangsloch (110) des Transformatorkerns (106) eingeführt zu werden.

## Revendications

1. Système de prothèse auditive implantable, comprenant :
- une unité implantable (102) configurée pour être au moins partiellement implantée dans le corps d'un utilisateur ;
- un noyau de transformateur (106) configuré pour être agencé au moins partiellement sous la peau de l'utilisateur, dans lequel le noyau de transformateur (106) est configuré pour être implanté complètement ou partiellement à l'intérieur d'une partie d'une oreille (162) de l'utilisateur ;
- un câblage interne (114) configuré pour connecter l'unité implantable (102) au noyau de transformateur (106), dans lequel le câblage interne (114) comprend un premier bobinage (113) autour du noyau de transformateur (106) ; et
- une unité externe (104) configurée pour être au moins partiellement agencée à l'extérieur du corps de l'utilisateur, dans lequel l'unité externe (104) est une prothèse auditive derrière l'oreille, une prothèse auditive dans l'oreille ou une prothèse auditive à crochet d'oreille, l'unité externe (104) comprenant :
- un logement (156) et un crochet d'oreille (158) fixé au logement (156) ;
- une circuiterie d'alimentation électrique (116) et
- un connecteur (118) couplé à la circuiterie d'alimentation électrique (116) configuré pour alimenter du courant électrique de la circuiterie d'alimentation électrique (116) à l'unité implantable (102) par le biais du noyau de transformateur (106), dans lequel le connecteur (118) comprend une première pièce de connecteur (122) et une seconde pièce de connecteur (124) ;
dans lequel le système comprend en outre :
- un premier chemin conducteur (128) connectant la circuiterie d'alimentation électrique (116) et la première partie de connecteur (122) de manière conductrice ;
- un second bobinage (115) peut être formé autour du noyau de transformateur (106) en joignant la première partie de connecteur (122) et la seconde partie de connecteur (124) ;
**caractérisé en ce que** le système comprend en outre :
- un chemin conducteur central en boucle (134) reliant une première section (136) de la seconde partie de connecteur (124) et une seconde section (138) de la seconde partie de connecteur (124) de manière conductrice, dans lequel le chemin conducteur central en boucle (134) s'étend de préférence au moins partiellement dans la première partie de connecteur (122) ;
- un chemin conducteur final (144) connectant la première partie de connecteur (122) et la circuiterie d'alimentation électrique (116) de manière conductrice ; et
- la première partie de connecteur (122) et la seconde partie de connecteur (124) peuvent être jointes pour connecter en série et de manière conductrice le premier chemin conducteur (128) et le chemin conducteur final (144) par le biais du chemin conducteur central en boucle (134).

2. Système de prothèse auditive implantable selon la revendication 1, comprenant en outre :
- un deuxième chemin conducteur (130) reliant le premier chemin conducteur (128) et la seconde partie de connecteur (124) de manière conductrice ;
- un premier chemin conducteur en boucle (132) reliant une première section (148) de la première partie de connecteur (122) et une seconde section (150) de la première partie de connecteur (122) de manière conductrice ;
- un chemin conducteur final en boucle (140) connectant une troisième section (152) de la première partie de connecteur (122) et une quatrième section (154) de la première partie de connecteur (122) de manière conductrice ; et
- un troisième chemin conducteur (142) reliant la seconde partie de connecteur (124) au chemin conducteur final en boucle (144) de manière conductrice.

3. Système de prothèse auditive implantable selon la revendication 2, dans lequel la première partie de connecteur (122) et la seconde partie de connecteur (124) peuvent être jointes pour relier en série et de manière conductrice le deuxième chemin conducteur (130) et le chemin conducteur central en boucle (134) par le biais du premier chemin conducteur en boucle (132) et pour connecter en série et de manière conductrice le chemin conducteur central en boucle (134) et le troisième chemin conducteur (142) par le biais du chemin conducteur final en boucle (140).

4. Système de prothèse auditive implantable selon la revendication 2 ou 3, dans lequel le premier chemin conducteur en boucle (132) et le chemin conducteur final en boucle (140) s'étendent uniquement dans la première partie de connecteur (122).

5. Système de prothèse auditive implantable selon l'une quelconque des revendications 1 à 4, dans lequel le premier chemin conducteur (128) et le chemin conducteur final (144) s'étendent uniquement dans la première partie de connecteur (122).

6. Système de prothèse auditive implantable selon l'une quelconque des revendications 1 à 5, dans lequel la première partie de connecteur (122) et/ou la seconde partie de connecteur (124) peuvent être jointes par une connexion par fiche.

7. Système de prothèse auditive implantable selon l'une quelconque des revendications 1 à 6, dans lequel les parties des chemins conducteurs (130, 134, 142) s'étendant entre la première partie de connecteur (122) et la seconde partie de connecteur (124) sont enchâssées au moins partiellement par un logement protecteur (126).

8. Système de prothèse auditive implantable selon l'une quelconque des revendications 1 à 7, dans lequel le noyau de transformateur (106) est essentiellement circulaire et/ou comprend un trou traversant (110).

9. Système de prothèse auditive implantable selon l'une quelconque des revendications 1 à 8, dans lequel l'unité implantable (102) est configurée pour générer une sortie et est sélectionnée parmi un groupe consistant d'un ou plusieurs de :
- un implant cochléaire comprenant une rangée d'électrodes implantables configurées pour être positionnées à l'intérieur d'une cochlée de l'utilisateur, la rangée d'électrodes étant configurée pour fournir des charges électriques en fonction de la sortie,
- un implant transmodiolaire auditif comprenant une rangée d'électrodes implantables configurées pour être positionnées à l'intérieur d'un modiolus de l'utilisateur, la rangée d'électrodes étant configurée pour fournir des charges électriques en fonction de la sortie,
- un implant de tronc cérébral auditif comprenant une rangée d'électrodes implantables configurées pour être implantées directement sur le tronc cérébral, la rangée d'électrodes étant configurée pour fournir des charges électriques en fonction de la sortie,
- une prothèse auditive à conduction osseuse comprenant un vibrateur implantable configuré pour être fixé sur le crâne de l'utilisateur, le vibrateur étant configuré pour générer des vibrations en fonction de la sortie, et
- un implant de l'oreille moyenne comprenant une unité vibratoire configurée pour se fixer à un des os de l'oreille moyenne et/ou à une des fenêtres de la cochlée, l'unité vibratoire étant configurée pour générer des vibrations en fonction de la sortie.

10. Système de prothèse auditive implantable selon l'une quelconque des revendications 1 à 9, dans lequel la circuiterie d'alimentation électrique (116) est agencée à l'intérieur du logement (126).

11. Système de prothèse auditive implantable selon la revendication 9 ou 10, dans lequel la première partie de connecteur (122) est au moins partiellement agencée à l'intérieur du crochet d'oreille (158) et dans lequel le crochet d'oreille (158) comprend un port (160), en particulier un port à fiche, pour connecter la première partie de connecteur (122) et la seconde partie de connecteur (124).

12. Système de prothèse auditive implantable selon la revendication 8 et 11, dans lequel la seconde partie de connecteur (124) est au moins partiellement agencée à l'intérieur du crochet d'oreille (158) et dans lequel au moins une partie du crochet d'oreille (158) est configurée pour être insérée à travers le trou traversant (110) du noyau de transformateur (106).
